# DEMANDE DE BREVET EUROPEEN

(11) **EP 1 122 249 A1**
(43) Date de publication de la demande: **08.08.2001**
(21) Numéro de dépôt: 00200344.0
(22) Date de dépôt: 02.02.2000
(51) Int. Cl.: C07D 301/12, C07D 303/04

(54) **Procédé de fabrication d'un oxiranne**

(71) Demandeur: SOLVAY (Société Anonyme), 1050 Bruxelles (BE)
(72) Inventeur: Strebelle, Michel, 1150 Bruxelles (BE); Catinat, Jean-Pierre, 7131 Waudrez (BE)
(74) Mandataire: Vande Gucht, Anne

(57) **Abrégé**

Procédé de fabrication d'un oxiranne par réaction entre une oléfine et du peroxyde d'hydrogène en présence d'un catalyseur et d'un diluant organique, selon lequel le peroxyde d'hydrogène est une solution aqueuse de peroxyde d'hydrogène obtenue par extraction avec de l'eau substantiellement pure du mélange issu de l'oxydation d'au moins une alkylanthrahydroquinone, sans traitement ultérieur de lavage et/ou de purification.

## Description

L'invention concerne un procédé de fabrication d'un oxiranne par réaction entre une oléfine et le peroxyde d'hydrogène en présence d'un catalyseur et d'un diluant. Elle concerne plus particulièrement un procédé de fabrication de 1,2-époxypropane (ou oxyde de propylène) par réaction entre le propylène et le peroxyde d'hydrogène.

Il est connu de fabriquer de l'oxyde de propylène par époxydation de propylène au moyen de peroxyde d'hydrogène et en présence d'un catalyseur de type TS-1, comme décrit par exemple dans la demande de brevet EP 0 230 949.

Le peroxyde d'hydrogène utilisé est en général fortement épuré en impuretés organiques. Ainsi, les solutions brutes de peroxyde d'hydrogène (H₂O₂) provenant de l'extraction d'un mélange issu de l'oxydation d'au moins une alkylanthrahydroquinone, subissent généralement une ou plusieurs étapes de lavage, extraction et/ou distillation avant d'être commercialisées et/ou utilisées dans des procédés de synthèse. Ceci est en particulier le cas pour les solutions d'H₂O₂ utilisées pour la fabrication des oxirannes.

La demande de brevet EP 549013 concerne un procédé intégré d'oxydation de substrats organiques et de production d'H₂O₂ par un procédé aux alkylanthraquinones (AO), qui utilise comme solvant d'extraction de l'H₂O₂ de la navette quinonique, le mélange eau/alcool utilisé lors de l'oxydation du substrat organique. La demanderesse a constaté que ce procédé présente plusieurs inconvénients :
- le manque de souplesse du procédé global suite à la dépendance de chaque étape de la synthèse (AO et oxydation) vis à vis de l'autre ;
- la limitation de la teneur en alcool des mélanges eau/alcool imposée par les conditions d'extraction, qui pénalise le taux de conversion de l'H₂O₂ lors de la réaction d'époxydation;
- les difficultés de séparation de phases lors de l'extraction avec un mélange eau/alcool ;
- le passage de quantités importantes de méthanol dans la navette des quinones d'où, compte tenu du faible point éclair du méthanol, un risque d'explosion non négligeable en phase vapeur lors de l'étape d'oxydation à la synthèse de l'H₂O₂;
- une quantité importante de quinones extraite dans le mélange eau/alcool, ce qui pénalise la rentabilité d'une installation industrielle; et
- la pollution de la navette quinonique par des sous-produits de la réaction d'oxydation.

La présente invention a pour objet un procédé de fabrication d'un oxiranne qui ne présente pas les inconvénients précités, tout en présentant un taux de conversion accru ainsi qu'une meilleure sélectivité que celle obtenue en utilisant un extrait épuré.

L'invention concerne dès lors un procédé de fabrication d'un oxiranne par réaction entre une oléfine et du peroxyde d'hydrogène en présence d'un catalyseur et d'un diluant organique, selon lequel le peroxyde d'hydrogène est une solution aqueuse de peroxyde d'hydrogène obtenue par extraction avec de l'eau substantiellement pure du mélange issu de l'oxydation d'au moins une alkylanthrahydroquinone, sans traitement ultérieur de lavage et/ou de purification.

La demanderesse a en effet constaté que, de manière surprenante, le fait d'utiliser pour la réaction d'époxydation, une solution d'H₂O₂ extraite avec de l'eau et non avec un mélange eau/alcool permet d'augmenter le taux de conversion de cet H₂O₂. En outre, le fait d'utiliser un extrait non épuré permet d'obtenir un gain de sélectivité par rapport à l'utilisation d'un extrait épuré.

Les procédés de production de peroxyde d'hydrogène utilisant les alkylanthraquinone(s) ou procédés AO sont bien connus et largement documentés dans la littérature (voir par exemple « Ullmann's Encyclopedia of Industrial Chemistry, Fifth Edition, 1989, Volume 3, p.447-57 »). Ils consistent à soumettre à une étape d'hydrogénation une solution de travail d'au moins une alkylanthraquinone et/ou d'au moins une tétrahydroalkylanthraquinone, dans un diluant pour produire une ou plusieurs alkylanthrahydroquinones et/ou alkyltétrahydroanthrahydroquinones. La solution de travail sortant de l'étape d'hydrogénation est ensuite soumise à une oxydation au moyen d'oxygène, d'air ou d'air enrichi en oxygène pour fournir du peroxyde d'hydrogène et reformer les alkylanthraquinones et/ou alkyltétrahydroanthraquinones. Le peroxyde d'hydrogène formé est ensuite séparé de la solution de travail au moyen d'une étape d'extraction. Selon la présente invention, cette extraction se fait au moyen d'eau substantiellement pure. La solution de travail sortant de l'étape d'extraction est ensuite recyclée à l'étape d'hydrogénation afin de recommencer le cycle de production de peroxyde d'hydrogène.

Par alkylanthraquinones, on entend désigner les 9,10-anthraquinones substituées par au moins une chaîne latérale alkyle de type aliphatique linéaire ou ramifiée comprenant au moins un atome de carbone. Habituellement, ces chaînes alkyles comportent moins de 9 atomes de carbone et, de préférence, moins de 6 atomes de carbone. Des exemples de telles alkylanthraquinones sont la 2-éthylanthraquinone, la 2-isopropyl-anthraquinone, les 2-sec- et 2-tert-butylanthraquinones, les 1,3-, 2,3-, 1,4- et 2,7-diméthylanthraquinones, les 2-iso-et 2-tert-amylanthraquinones et les mélanges de ces quinones.

Par eau substantiellement pure, on entend désigner une eau contenant moins de 3% de diluants organiques, en particulier d'alcool(s), de préférence moins de 0.1%, voire moins de 0.001% de ces diluants. L'eau d'extraction peut toutefois avantageusement contenir des substances inorganiques à raison de 0.001 % minimum, de préférence 0.005%, voire 0.01% minimum. La teneur en substances inorganiques ne dépassera toutefois pas 1%, de préférence 0.5%, voire 0.1%. Ces substances inorganiques sont avantageusement des substances ayant un effet régulateur de pH, tels que les acides, et en particulier, les acides forts tels que l'acide nitrique, l'acide phosphorique, ou les sels de tels acides. Ces substances inorganiques peuvent tout aussi avantageusement être des substances ayant un effet stabilisant sur l'H₂O₂ tel que les sels de métaux alcalins ou alcalino-terreux, et en particulier, de sodium, tel que le pyrophosphate de sodium. La solution d'extraction peut donc comprendre des cations métalliques (tel que des métaux alcalins ou alcalino-terreux, comme le sodium) et/ou des anions tels que les phosphates, nitrates ... en des teneurs faibles, généralement inférieures à 10 g/l, mais supérieures à 0.01 g/l.

La solution d'H₂O₂ issue de l'extraction, ou solution d'H₂O₂ brute, contient généralement moins de 50% d'H₂O₂, plus souvent moins de 40% d'H₂O₂. Elle contient généralement plus de 5% d'H₂O₂, le plus souvent plus de 10%, voire plus de 30%. Elle ne subit aucun traitement ultérieur de lavage et/ou de purification avant sa mise en oeuvre dans la réaction d'époxydation. En conséquence, elle contient des impuretés organiques (produits de dégradation de la navette quinonique) et inorganiques (cations et anions introduits par l'eau d'extraction, ainsi que ceux déjà présents dans le mélange issu de l'oxydation de la ou des alkylanthrahydroquinones). La solution issue de l'extraction peut donc comprendre des impuretés organiques exprimées en COT (concentration en carbone organique total), défini selon la norme ISO 8245, à raison d'au moins 0.001 g/l, voire au moins 0.01 g/l, ou même au moins 0.1 g/l, mais pas plus de 10 g/l, voire 1 g/l, ou même 0.2 g/l. Elle peut également contenir des cations métalliques (tel que des métaux alcalins ou alcalino-terreux, comme le sodium) et/ou des anions tels que les phosphates, nitrates ... en des teneurs faibles, généralement inférieures à 10 g/l, mais supérieures à 0.01 g/l.

Avant son utilisation dans la réaction d'époxydation, la solution d'H₂O₂ brute peut être diluée avec de l'eau ou tout autre solvant ou diluant liquide n'ayant pas d'influence négative sur la réaction d'époxydation. En général, la solution aqueuse utilisée pour l'époxydation contient au moins 10 % en poids d'H₂O₂, en particulier au moins 20 % en poids. Elle contient le plus souvent au maximum 50% en poids de composé peroxydé, en particulier 40% en poids.

L'oxiranne qui peut être préparé par le procédé selon l'invention est un composé organique comprenant un groupement répondant à la formule générale :

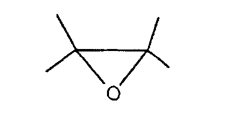

L'oxiranne contient généralement de 3 à 10 atomes de carbone, de préférence de 3 à 6 atomes de carbone. Un oxiranne qui peut être préparé de manière avantageuse par le procédé selon l'invention est le 1,2-époxypropane.

Les oléfines qui conviennent bien dans le procédé selon l'invention contiennent généralement de 3 à 10 atomes de carbone et de manière préférée, 3 à 6 atomes de carbone. Le propylène et le butylène conviennent particulièrement. Le propylène est préféré.

L'oléfine selon la présente invention peut contenir de l'ordre de 30 à 50%, voire même jusque 95%, d'un ou de plusieurs alcanes. Il est par contre déconseillé d'utiliser des fluides contenant plus de 95% d'alcane(s), et il est même préférable d'utiliser des fluides ne contenant pas plus de 85% d'alcane(s). Le ou les alcanes contenus dans le fluide selon la présente invention contiennent généralement de 3 à 10 atomes de carbone et de manière préférée, 3 à 6 atomes de carbone. Dans le cas où l'oléfine selon l'invention est le propylène, le ou les alcanes sont majoritairement constitués de propane.

Les catalyseurs utilisés dans le procédé selon l'invention contiennent avantageusement une zéolite, à savoir un solide contenant de la silice qui présente une structure cristalline microporeuse. La zéolite est avantageusement exempte d'aluminium. Elle contient de préférence du titane.

La zéolite utilisable dans le procédé selon l'invention peut avoir une structure cristalline de type ZSM-5, ZSM-11, MCM-41 ou de type zéolite bêta. Les zéolites de type ZSM-5 conviennent bien. Celles présentant une bande d'adsorption infrarouge à environ 950-960 cm⁻¹ sont préférées.

Les zéolites qui conviennent particulièrement bien sont les silicalites au titane. Celles répondant à la formule xTiO₂(1-x)SiO₂ dans laquelle x est de 0,0001 à 0,5, de préférence de 0,001 à 0,05 sont performantes. Des matériaux de ce type, connus sous le nom de TS-1 et présentant une structure cristalline de type ZSM-5, donnent des résultats particulièrement favorables.

Le milieu réactionnel selon l'invention comprend généralement une phase liquide et une phase gazeuse.

Les diluants organiques utilisables dans le procédé selon l'invention peuvent être des dérivés organiques tels que les alcools aliphatiques, contenant de 1 à 4 atomes de carbone. On peut citer à titre d'exemple le méthanol. La teneur en diluant de la phase liquide du milieu réactionnel est avantageusement supérieure à 35%, préférablement supérieure à 60%, voire 75%. La teneur en diluant de la phase liquide du milieu réactionnel est toutefois généralement inférieure à 99%, préférablement inférieure 95%.

Dans une variante préférée du procédé selon l'invention, on peut séparer l'oxiranne produit dans le milieu réactionnel par extraction liquide-liquide avec un solvant tel que décrit dans la demande de brevet WO 99/14208 au nom de la demanderesse.

Le procédé selon l'invention peut être continu ou discontinu. S'il est continu, l'oléfine n'ayant pas réagi peut être recyclée vers le réacteur.

Le réacteur dans lequel se déroule le procédé selon l'invention peut être alimenté par une solution provenant directement de l'étape d'extraction aqueuse d'un procédé AO. Dans ce cas, l'installation dans laquelle se déroule le procédé selon l'invention intègre également une installation de fabrication de la solution d'H₂O₂ selon un procédé AO. Une telle installation et un procédé l'utilisant constituent également un objet de la présente invention.

Alternativement, la solution peut été stockée et/ou véhiculée avant son alimentation au réacteur, ce qui est le cas des solutions purifiées utilisées à l'heure actuelle.

Dans le procédé selon l'invention, un gaz n'ayant pas d'influence négative sur la réaction d'époxydation peut également être alimenté au réacteur. En effet, dans la demande de brevet WO 99/48883, la demanderesse a trouvé qu'en introduisant un composé gazeux dans le milieu réactionnel à un débit suffisant pour permettre d'entraîner l'oxiranne produit et de le sortir du réacteur en même temps que le composé gazeux, on diminue le temps de contact entre l'oxiranne produit et le milieu réactionnel d'époxydation. On évite ainsi la formation des sous-produits et on augmente la sélectivité de l'époxydation.

Une forme de réalisation avantageuse du procédé selon l'invention consiste à introduire la phase gazeuse dans le réacteur à un débit tel qu'il permet non seulement d'entraîner au moins une partie de l'oxiranne produit mais également de faire circuler la phase liquide dans le réacteur, en particulier lorsque celui-ci est un réacteur de type boucle. Dans ce cas, la phase gazeuse est généralement introduite dans le réacteur à un débit tel que le rapport molaire du débit de cette phase gazeuse au débit d'alimentation de l'H₂O₂ soit d'au moins 5, en particulier d'au moins 8, les valeurs d'au moins 10 étant courantes. Le rapport molaire de ces débits est généralement inférieur ou égal à 100, en particulier à 60, les valeurs inférieures ou égales à 40, et même 20, étant courantes.

Dans le procédé selon l'invention on peut utiliser tout type de réacteur, en particulier un réacteur de type boucle. Les réacteurs de type boucle à bullosiphon, dans lesquels la circulation du liquide et aussi éventuellement du catalyseur est obtenue par barbotage d'un gaz dans l'une des branches, conviennent bien. Ce type de réacteur est décrit dans la demande de brevet WO 99/48883 mentionnée ci-dessus.

Dans le procédé selon l'invention il peut s'avérer intéressant de maintenir le pH de la phase liquide lors de la réaction entre l'oléfine et l'H₂O₂ à une valeur d'au moins 4,8, en particulier d'au moins 5. Le pH est avantageusement inférieur ou égal à 6,5, en particulier à 6. De bons résultats sont obtenus lorsque le pH est de 4,8 à 6,5, de préférence de 5 à 6. Le pH de la phase liquide lors de la réaction d'époxydation peut être contrôlé par addition d'une base. Cette base peut être choisie parmi les bases solubles dans l'eau. Il peut s'agir de bases fortes. On peut citer à titre d'exemples de bases fortes NaOH et KOH. Il peut également s'agir de bases faibles. Les bases faibles peuvent être inorganiques. On peut citer à titre d'exemples de bases faibles inorganiques NH₄OH, Na₂CO₃, NaHCO₃, Na₂HPO₄, K₂CO₃, Li₂CO₃, KHCO₃, LiHCO₃, K₂HPO₄. Les bases faibles peuvent aussi être organiques. Des bases faibles organiques qui peuvent convenir sont les sels de métaux alcalins ou alcalino-terreux d'acides carboxyliques contenant de préférence de 1 à 10 atomes de carbone. On peut citer à titre d'exemple l'acétate de sodium. Les bases faibles donnent de bons résultats. Les bases faibles organiques sont préférées. L'acétate de sodium convient particulièrement bien.

Le rapport molaire entre la quantité d'oléfine engagée et la quantité d'H₂O₂ engagée est généralement supérieur ou égal à 0.1, en particulier supérieur ou égal à 1, et de préférence supérieur à 5. Ce rapport molaire est le plus souvent inférieur ou égal à 100, en particulier inférieur ou égal à 50 et de préférence inférieur ou égal à 25.

Dans le procédé selon l'invention, lorsqu'il est réalisé en continu et en présence d'une zéolite, l'H₂O₂ est généralement mis en oeuvre en une quantité d'au moins 0,005 mole par heure et par gramme de zéolite, en particulier, d'au moins 0,01 mole par heure et par gramme de zéolite. La quantité d'H₂O₂ est habituellement inférieure ou égale à 2,5 moles par heure et par gramme de zéolite et, en particulier, inférieure ou égale à 1 mole par heure et par gramme de zéolite. Une préférence est montrée pour une quantité d'H₂O₂ supérieure ou égale à 0,03 mole par heure et par gramme de zéolite et inférieure ou égale à 1 mole par heure et par gramme de zéolite.

La réaction entre l'oléfine et l'H₂O₂ peut s'effectuer en présence d'un sel tel qu'un sel de métal ou un sel d'ammonium. Le métal peut être choisi parmi les métaux alcalins et alcalino-terreux tels que le lithium, le sodium, le potassium, le césium, le magnésium, le calcium, le strontium et le baryum. Les sels de métaux sont avantageusement les halogénures, les oxydes, les hydroxydes, les carbonates, les sulfates, les phosphates et les sels d'acides organiques tels que les acétates. Les halogénures sont généralement les fluorures, les chlorures, les bromures et les iodures. Une préférence est montrée pour les chlorures. Le sel avantageusement engagé dans le procédé selon la présente invention est de préférence un halogénure de métal alcalin et, avantageusement, du chlorure de sodium. La quantité de sel de métal mise en oeuvre est exprimée comme la teneur en ions métalliques ou d'ammonium provenant du sel par rapport à la quantité de catalyseur exprimée en millimoles (mmol) de métal ou d'ammonium par gramme de zéolite. Cette teneur peut être supérieure ou égale à 10⁻⁴ mmol/g de zéolite et inférieure ou égale à 10 mmol/g de zéolite. Avantageusement, la teneur en sel de métal est supérieure ou égale à 10⁻³ mmol/g de zéolite et inférieure ou égale à 1 mmol/g de zéolite. Une préférence est montrée pour une teneur supérieure ou égale à 10⁻² mmol/g de zéolite et inférieure ou égale à 0,5 mmol/g de zéolite.

La température de la réaction entre l'oléfine et l'H₂O₂ est avantageusement supérieure à 35 °C pour remédier à la désactivation progressive du catalyseur . Il est avantageux de réaliser la réaction à une température supérieure ou égale à 40°C et de préférence supérieure ou égale à 45°C. Une température supérieure ou égale à 50°C est tout particulièrement préférée. Toutefois, la température de réaction est généralement inférieure à 100 °C et de préférence, inférieure à 80°C. La température à laquelle l'oléfine réagit avec l'H₂O₂ est généralement comprise entre 40°C et 100°C, et de préférence comprise entre 45°C et 80°C.

Dans le procédé selon l'invention, la réaction entre l'oléfine et l'H₂O₂ peut avoir lieu à pression atmosphérique. Elle peut également se dérouler sous pression. Généralement, cette pression n'excède pas 40 bar. Une pression de 20 bar convient bien en pratique.

### Exemples 1 (selon l'invention) et 2C (comparatif)

Un réacteur continu contenant 5.25 g de TS-1 est maintenu à 35°C à pression atmosphérique et alimenté par 0.57 mol d'H₂O₂/h, introduite à l'état de solution aqueuse à 40 % poids, par 475 ml de méthanol/h ainsi que par 250 IN/l (soit 11.2 mol/h) de propylène. Les phases liquides et gazeuses sortantes sont analysées pour déterminer les proportions des différents produits organiques ainsi que le taux de conversion de l'H₂O₂.

Le tableau ci-dessous reprend les résultats obtenus à l'issue d'essais au départ d'un catalyseur TS-1 frais préparé suivant les recettes connues dans la littérature.

| Exemple n° | 1 (invention) | 2C (comparaison) |
|---|---|---|
| | H₂O₂ brute d'extraction | H₂O₂ épurée |
| Taux de conversion de l'H₂O₂ après 2 h de marche | 76.7 | 76.0 |
| Idem après 6 h | 54 | 53 |
| Sélectivité* après 6 h | 90.5 | 85.4 |

| | | |
|---|---|---|
| * la sélectivité est exprimée par le rapport mol/mol OP(oxyde de propylène) formé/total de produits organiques formés | | |

On observe, comme connu, une perte progressive de l'activité du catalyseur qui n'est pas affectée par la qualité d'H₂O₂ mise en oeuvre. Seule la sélectivité est favorablement influencée en présence de l'H₂O₂ brute.

Il convient de noter les teneurs respectives en anions et cations de ces solutions d'H₂O₂:

| Teneur en mg/l | H₂O₂ brute | H₂O₂ épurée |
|---|---|---|
| Na | 26 | 2.3 |
| autres cations (hors H+) | <0.3 | <0.3 |
| NO₃ | 34 | 3.7 |
| Phosphates exprimés en P | 28 | 1.4 |
| TOC | 172 | 69 |

### Exemples 3 (selon l'invention) et 4C (comparatif)

Le tableau suivant reprend des essais en tout point semblables à des exemples 1 et 2C, lors d'un cycle suivant à l'issue de la régénération du catalyseur. Cette régénération est obtenue par passage d'air chauffé à 300°C pendant 7h sur le catalyseur.

| Exemple n° | 3 (invention) | 4 C (comparaison) |
|---|---|---|
| | H₂O₂ brute | H₂O₂ épurée |
| Taux de conversion de l'H₂O₂ après 2 h de marche | 75.4 | 75.6 |
| Idem après 6 h | 53.5 | 54.3 |
| Sélectivité** après 6 h | 91.1 | 85.7 |

On confirme que les activités sont, à la précision des mesures près, restées identiques et que l'écart de sélectivité se maintient.

### Exemples 5C (comparatif) et 6 (selon l'invention)

Une solution de synthèse d'H₂O₂ obtenue après oxydation d'une navette de quinones/hydroquinones a été extraite à l'aide d'un mélange méthanol/eau à 52 % poids de méthanol . Cet extrait aqueux a ensuite été utilisé dans un essai d'époxydation du propylène (exemple 5C) et les performances obtenues ont été comparées à celle d'un essai similaire effectué avec de l'H₂O₂ brute 40 % poids dans l'eau, provenant de l'extraction de la même navette avec de l'eau substantiellement pure (exemple 6). Cette navette contient 11.8 g/kg d'H₂O₂.

L'extraction avec le mélange eau/alcool a été effectuée en 4 étapes : Une 1ère extraction a été effectuée en traitant 14331 g de navette (contenant 169.1 g d'H₂O₂ au total) par 1511 g du mélange méthanol/eau. La phase méthanol-eau est plus dense que la solution organique de départ et décante relativement rapidement (en 15 min environ) pour donner 1085 g d'extrait. Sa concentration en H2O2 déterminée par iodométrie est égale à 3.18 mol H₂O₂/kg, ce qui correspond à 3.45 mol ou 117.4 g d'H2O2 (= 69 % du total présent).

Une 2e extraction a été effectuée avec 1522 g du même mélange méthanol/eau. La séparation est moins évidente. La décantation est assez lente : plus d'1 h est nécessaire pour pouvoir procéder à la séparation des phases. Contrairement à la 1ère extraction, la phase méthanol-eau est cette fois la moins dense et consiste en 1215 g d'extrait. Sa concentration en H₂O₂ est égale à 0.833 mol/kg, ce qui équivaut à 1.012 mol ou 34.4 g d'H₂O₂. On a donc récupéré en deux extractions 90 % de l'H₂O₂ total.

Une 3e extraction a été effectuée avec 1511 g du même mélange méthanol/eau. Même difficulté de séparation, avec récupération d'environ 1446 g de phase méthanol-eau. Sa concentration en H₂O₂ est égale à 0.244 mol/kg, ce qui équivaut à 0.353 mol ou 12.0 g d'H₂O₂ (soit en 3 extractions, 96.9 % de l'H₂O₂ total).

Enfin, une 4e extraction a été effectuée avec 1517 g du même mélange méthanol/eau. Même difficulté de séparation, avec récupération d'environ 1497 g de phase méthanol-eau. Sa concentration en H₂O₂ est égale à 0.071 mol/kg, ce qui équivaut à 0.106 mol ou 3.6 g d'H₂O₂ (soit en 4 extractions, 99.0 % de l'H₂O₂ total).

Les 4 extraits ont ensuite été mélangés, ce qui conduit à une solution méthanol/eau à 0.94 mol H₂O₂/kg (effectivement vérifié par titrage). La teneur en méthanol déterminée par CPV est voisine de 437 g/kg.

La teneur en quinones "utiles"(= pouvant servir à produire de l'H₂O₂) perdue dans cette phase est de 0.020 g/kg d'extrait.

Il y a par ailleurs manifestement passage d'une partie du méthanol dans la navette de quinones, ainsi que le démontrent les écarts entre les poids des mélanges méthanol/eau mis en oeuvre et ceux des extraits recueillis (en particulier pour les 1ère et 2e extractions). La teneur en méthanol de la navette de quinones, déterminée par CPV, est effectivement voisine de 6.0 % poids.

Les essais d'époxydation du propylène (Pe) ont été effectués dans une installation de type bullosiphon dans les conditions suivantes : T : 55 °C; débit de Pe: 75 IN/h; H₂O₂ : 0.17 mol H₂O₂/h; concentration en H₂O₂ dans la boucle à conversion nulle : 1.0 mol/kg; catalyseur : 0.53 g de TS-1.

En ce qui concerne l'exemple 5, l'introduction du seul mélange des quatre extraits méthanol/eau contenant l'H₂O₂ dans l'installation de type bullosiphon conduirait, suite au stripping, à un milieu "pauvre" en méthanol (conc. < 440 g/kg). Dès lors, un complément de méthanol a été rajouté de manière à maintenir sa concentration dans la boucle à ≈ 440 g/kg, ce qui correspond à la teneur en méthanol de l'essai de référence à l'H₂O₂ brute (exemple 6).

Les résultats obtenus figurent dans le tableau ci-dessous :

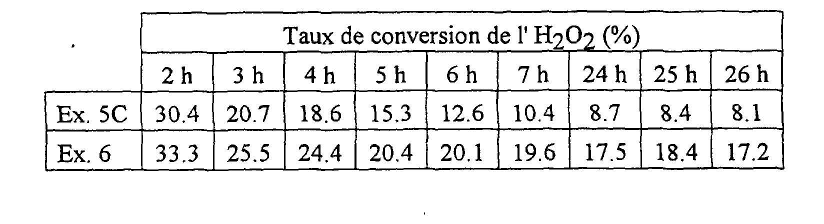

## Revendications

1. Procédé de fabrication d'un oxiranne par réaction entre une oléfine et du peroxyde d'hydrogène en présence d'un catalyseur et d'un diluant organique, selon lequel le peroxyde d'hydrogène est une solution aqueuse de peroxyde d'hydrogène obtenue par extraction avec de l'eau substantiellement pure du mélange issu de l'oxydation d'au moins une alkylanthrahydroquinone, sans traitement ultérieur de lavage et/ou de purification.

2. Procédé selon la revendication 1, dans lequel l'oxiranne est le 1,2-époxypropane et l'oléfine est le propylène.

3. Procédé selon la revendication 1 ou 2, dans lequel l'eau d'extraction contient moins de 3% en diluants organiques, en particulier d'alcool(s).

4. Procédé selon l'une quelconque des revendications précédentes, dans lequel la solution d'H₂O₂ obtenue par extraction contient entre 0.001 g/l et 10 g/l d'impuretés organiques exprimées en COT.

5. Procédé selon l'une quelconque des revendications précédentes, dans lequel la solution d'H₂O₂ obtenue par extraction contient des cations métalliques (tel que des métaux alcalins ou alcalino-terreux, comme le sodium) et des anions (tels que les phosphates, nitrates) en des teneurs comprises entre 0.01 g/l et 10 g/1.

6. Procédé selon l'une quelconque des revendications précédentes, dans lequel la solution d'H₂O₂ obtenue par extraction comprend entre environ 5% et 50% de peroxyde d'hydrogène.

7. Procédé selon l'une quelconque des revendications précédentes, dans lequel le catalyseur est du silicalite au titane, de préférence de type TS-1 présentant une structure cristalline de type ZSM-5, et le diluant est le méthanol.

8. Procédé selon l'une quelconque des revendications précédentes, dans lequel le milieu réactionnel comprend une phase liquide et une phase gazeuse, et dans lequel la teneur en diluant organique de la phase liquide est supérieure à 35%.

9. Installation permettant la mise en oeuvre d'un procédé selon les revendications 1 à 8, qui intègre une installation de fabrication de la solution d'H₂O₂ selon un procédé AO.

10. Procédé intégré de fabrication d'H₂O₂ selon un procédé AO et de fabrication d'oxiranne par réaction entre une oléfine et l'H₂O₂, utilisant l'installation selon la revendication 9.
